# EUROPEAN PATENT SPECIFICATION

(11) **EP 3 287 148 B1**
(45) Date of publication and mention of the grant of the patent: **25.10.2023**
(21) Application number: 17188038.8
(22) Date of filing: 25.08.2017
(51) Int. Cl.: A61L 2/22

(54) **SYSTEM FOR BACTERIA DETECTION AND ELIMINATION ACCORDING TO SID**
SYSTEM FÜR BAKTERIENNACHWEIS UND -ENTFERNUNG GEMÄSS SID
SYSTÈME DE DÉTECTION ET ÉLIMINATION DE BACTÉRIES SELON SID

(30) Priority: 26.08.2016 NL 1042018
(43) Date of publication of application: 28.02.2018
(73) Proprietor: Bacsassin B.V., 2925 CM Krimpen aan den IJssel (NL)
(72) Inventor: FENTROSS, Rick, 2925 CM Krimpen aan den IJssel (NL); KRIJGER, Joris, 2311 CR Leiden (NL)
(74) Representative: Winger

(56) References cited:
- EP-A2- 1 421 855
- EP-A2- 2 835 647
- WO-A2-01/50872
- WO-A2-03/087772
- WO-A2-2015/093957
- US-A1- 2010 227 332
- CARLTON R M ET AL: "Bacteriophage P100 for control of Listeria monocytogenes in foods: Genome sequence, bioinformatic analyses, oral toxicity study, and application", REGULATORY TOXICOLOGY AND PHARMACOLOGY, ACADEMIC PRESS,NEW YORK, NY, US, vol. 43, no. 3, 1 December 2005 (2005-12-01), pages 301-312, XP027187759, ISSN: 0273-2300 [retrieved on 2005-11-09]

## Description

### Field and Background

The current disclosure forms a contribution to resolving the problem of the "ongoing explosion of antibiotic-resistant infections that continue to plague global [...] health care" (Spellberg et al., p. 1). Antimicrobial resistance, the resistance of a microorganism to an antimicrobial drug, endangers the efficacy of our present-day antibiotics. Drug-resistant microorganisms pose serious challenges on global health care and, due to the failure of standard treatment, result in prolonged illness and a greater risk of death. It is estimated by the WHO that infections caused by resistant microorganisms result in a death rate of over 50%. Most notoriously are MRSA (methicillin-resistant *Staphylococcus aureus*) infections of which it is estimated that people, once infected, are 64% more likely to die than people with a non-resistant form of the infection. In the US alone the Centre for Disease Control and Prevention (CDC) estimated a minimum number of illnesses and deaths caused by antibiotic resistance of at least 2.049.422 illnesses and 23.000 deaths per year.

Partially this antibiotic resistance crisis can be attributed to the overuse and misuse of these medications (Ventola, 2015). As van Geijlswijk et al. have shown the human antibiotic use remains stable around the 4 DDD (Defined Daily Dose) per year whereas in the veterinary sector the usage increased from 20 DDD to 30 DDD per year. This extreme antibiotic use enables multi-drug resistant (MDR) bacteria to spread. since animal wastes are used in organic fertiliser production the antibiotics used in the veterinary sector find their way into the soil, the plants and circulates in the rest of the agricultural processes. From here the MDR's can spread to humans.

Along with this there are the body's own bacteria (Klebsiella, Escherichia Coli) that can produce antibiotic-neutralizing enzymes; ESBL's (Extended Spectrum Beta-Lactamase). These enzymes neutralise the functioning components of a.o. penicillin's and cephalosporin's. Antimicrobial resistance is facilitated by the use of antibiotics. Inherent to antibiotic use is the disturbance of the immune system (dysbiosis) due to their killing of, along with the bacteria causing the illness, the good bacteria protecting the body from infection. This enables drug-resistant bacteria to grow.

The two facets of antibiotic overuse and resulting drug-resistance form an intricate healthcare problem globally. On the one hand the antimicrobial resistance should be lowered by diminishing the amount of antibiotics circulating in the agricultural sector. This will increase the chance of success of common antibiotics. On the other hand, if infection occurs that is characterised by antimicrobial resistance a method is required for specific bacteria elimination leaving the good bacteria intact.

This has now been realised by the revival of an old science: the research on bacteriophages. In the broadest definition bacteriophages are workable viruses which work within a short time frame on the annihilation of a specific group of bacteria and dissolve when the bacteria is whipped out. A phage functions as a sniper by very selectively targeting a specific bacteria that it eliminates. This way the substantial negative side effect of antibiotics, namely the disturbed balance in the autoregulation of the immune system (dysbiosis), can be overcome. To broaden its range of efficacy, when for example multiple pathogenic bacteria appear on a surface, it is possible to cluster phages in a mixture, a phage 'cocktail'. The effectivity of phages as safe antibacterial agents has been proven in studies ranging bioinformatics analysis of bacteriophage control of Listeria monocytogenes in foods (Carlton et al. 2005) to phages as an antibiotic alternative for resistant bacteria such as a.baumannii. In regard to the latter Lin et al. (2010) tested bacteriophages as antibiotic alternative for the A.baumannii bacterium and concluded that phages are "a good candidate as a therapeutic/disinfectant agent to control nosocomial infections caused by MDRAB". Even more, in 2015 Kitti et al. demonstrated the high efficacy of the bacteriophage cocktail on inhibiting the growth of A.baumannii. The potential for bacteriophages is high in several industries while the amount of applications are, to this date, limited.

So far, the main focus of known systems, e.g. as proposed by WO01/50872A2 and WO 2015/093957, was the use of a bacteriophage dispersing mechanism for sanitation purposes. However, adequate treatment of surfaces with bacteriophages requires more than an apt administration system: of equal importance is the determination of the bacterium present on the surface. To meet the full decontaminating potential of phage administration and to optimise the functioning of the phages, what is needed is an advanced discriminative detection method. To our knowledge the only system proposed with this combination of bacteria detection and phage decontamination is proposed in EP2835647A2 where biosensors are used for bacteria detection and phage vaporization for habitat decontamination. This system, however, knows two major limitations: it can only detect *one* fixed type of bacterium because the proposed biosensor detection means are not able to detect bacterial contamination after gene mutation has occurred. This means that the sensors are only able to detect the presence of a fixed genetic structure. Furthermore, the biosensors are static and can only be placed on non-biological surfaces (such as walls and tables). This limits habitat decontamination to preinstalled rooms, unsuitable for adequate action in case of an outbreak.

WO2015093957A2 discloses means and methods for selectively decontaminating a room for a specific bacterium. More in particular, it discloses means and methods for providing a room with a decontaminant comprising determining a bacterial species for which the room is to be decontaminated; preparing a decontaminant that comprises at least one bacteriophage (phage) that is known to propagate in bacteria of the bacterial species; releasing the decontaminant as an aerosol with droplets of an average diameter of 0.2 - 40 micrometer into the room; and incubating the room with the aerosol for a period of at least 1 minute. Further disclosed are means and methods for selectively decontaminating an individual with an aerosol.

WO0150872A2 discloses a method for produce sanitation using bacteriophages. According to one embodiment, the method includes the steps of (1) providing at least one bacteriophage; and (2) applying the bacteriophage to the produce. The produce may include fruits and vegetables. The produce may be freshly-cut produce, damaged produce, diseased produce, or contaminated produce. The produce may be sprayed with bacteriophage, washed with bacteriophage, immersed in a liquid containing bacteriophage, etc. The bacteriophage may be applied once, periodically or continuously. In one embodiment, chemical sanitizers may also be applied to the produce.

EP2835647A2 discloses a method and a device for decontaminating a habitat, comprising the following steps: a) specifically identifying at least one contamination species in the habitat; b) selecting a contamination species based on the identified specifically effective decontamination agent; c) distribution of the decontamination agent in the habitat to be decontaminated; and d) analysis of the decontamination status in the habitat.

### References:

Adan, A., Quintana, B., Vazquez, A.S., Olivares, A., Parra, E. Prieto, S. 2015. Towards the Automatic Scanning of Indoors with Robots. Sensors, Vol. 15.

Buzalewicz, I., Wieliczko, A., Podbielska, H. 2001. Influence of various growth conditions on Fresnel diffraction patterns of bacteria colonies examined in the optical system with converging spherical wave illumination.Optic Express, Vol. 19, No. 22.

Carlton RM, Noordman WH, Biswas B, de Meester ED, Loessner MJ.2005. Bacteriophage P100 for control of Listeria monocytogenes in foods: genome sequence, bioinformatic analyses, oral toxicity study, and application. Regulatory Toxicology and Pharmacology, 43, 3.

Congressional Research Service Report Life expectancy in the United States. Mar, 2005. Available at:http://www.cnie.org/nle/crsreports/05mar/RL32792.pdf. Accessed March 4th, 2016.

van Geijlswijk IM, Mevius DJ, Puister-Jansen LF. Kwantificeren van veterinair antibioticagebruik. Tijdschrift Diergeneeskunde 2009;134(2):69-73.

Hasman, H. , Saputra, D., Sicheritz-Ponten, T., Lund, O., Svendsen, C.A., Frimodt-Moller, N., Aarestrup, F.M. 2014. Rapid Whole-Genome Sequencing for Detection and Characterization of Microorganisms Directly from Clinical Samples. Journal of Clinical Microbiology. Vol. 52, no.1.

Kutter, E., De Vos, D., Gvasalia, G., Alavidze, Z., Gogokhia, L., Kuhl S., Abedon, ST. 2010. Phage therapy in clinical practice: treatment of human infections. Current Pharmaceutical Biotechnology. Vol 11, no.1.

Lin NT, Chiou PY, Chang KC, Chen LK, Lai MJ. 2010. Isolation and characterization of phi AB2: a novel bacteriophage of Acinetobacter baumannii, 161(4).

Kikki T, Thummeepak R, Leungtongkam U, Kunthalert D, Sutthirat S,. 2015. Efficacy of Acinetobacter baumannii bacteriphage cocktail on Acinetobacter baumannii growth. African Journal of Microbiological Research, 9, 42.

Spellberg B, Guidos R, Gilbert D, Bradley J, Boucher H, Scheld WM, Bartlett JG, and Edwards J. The epidemic of antibiotic-resistant infections: a call to action for the medical community from the Infectious Diseases Society of America. Clinical Infectious Diseases 2008; 46:155- 164. Ventola, C.L. 2015. The Antibiotic Resistance Crisis, Part 1: Causes and Threats. P&T. Vol 40, nr 4.

### Summary of the invention

The claimed invention is set out in the appended set of claims.

The current disclosure overcomes said limitations of existing systems by offering a system with flexible detection devices capable of detecting a wide range of bacteria subtypes without requiring pre-instalment in a habitat. This broadens the application scope of the disclosure. Since it is not limited to the decontamination of mere habitats its applications could range from production facilities to miniaturized use on patient skin examination.

The current disclosure provides a system that offers adequate and rapid bacteria detection by extending the phage-administrating device with two layers of highly advanced measurements. This forms a three-phase model wherein firstly, by means of spectral analysis a general scanning of the determined space is performed. This results in either a general warning or an indication of safety. In case traces of possibly pathogenic bacteria are found a second analysis is performed by means of miniaturized and portable DNA, RNA or mRNA sequencing techniques. In a fully automated embodiment samples are provided to the sequencing unit via a vacuum system that takes in water samples of the pre-humidified room. This results in an apt bacteria determination enabling the phage device to administer the correct phage or combination of phages. Each step in the process, from searching to elimination, can be operated manually but is designed in a way that operations can be performed automatic and without human interference being needed. In each phase results are communicated to a CPU system that translates data output into functional input for the new phase.

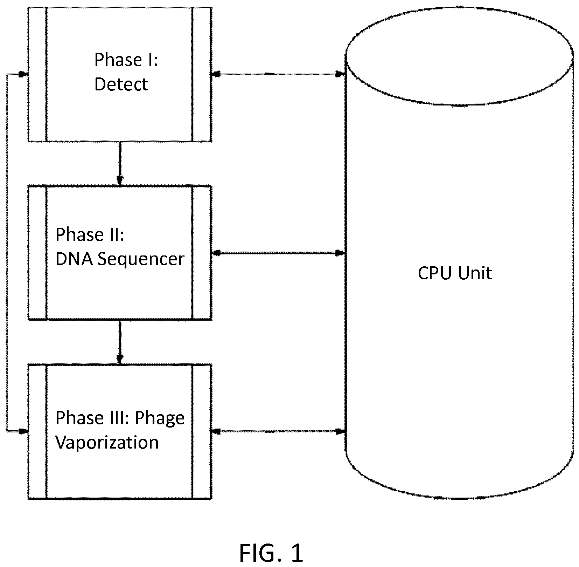

This way the current disclosure surpasses the existing methods and devices of bacteriophage sanitation, enabling rapid and highly accurate determination on various biological and non-biological surfaces without the requirement of pre-instalment. Due to the compact nature of both the sequencer and the scanning device the system offers far more mobility than fixed biosensors. By complementing the powerful method of bacteriophage decontamination with both an optical first warning system and an in depth determination of bacterial subtypes via genome-sequencing a unique combination of rapid, broad surface, bacteria detection and phage decontamination is developed, see Figure 1. The current disclosure therefore presents a method to actively search for microbiological contaminants on biological and non-biological surfaces, detect contaminants when present, identify them up to serotype level via DNA or genome sequencing and, by communicating these results to the phage vaporizer, enables the administrating device to match the phage to the found microbiological contaminant.

### Brief description of the drawings

FIG. 1 illustrates a flowchart showing exemplary embodiments of the loop for bacteria detection, identification and destruction, or Seek, Identify and Destroy (SID) loop.
FIG. 2 illustrates a flowchart showing and exemplary flow for a CPU unit

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed Description

### Searching, Detection and Determination

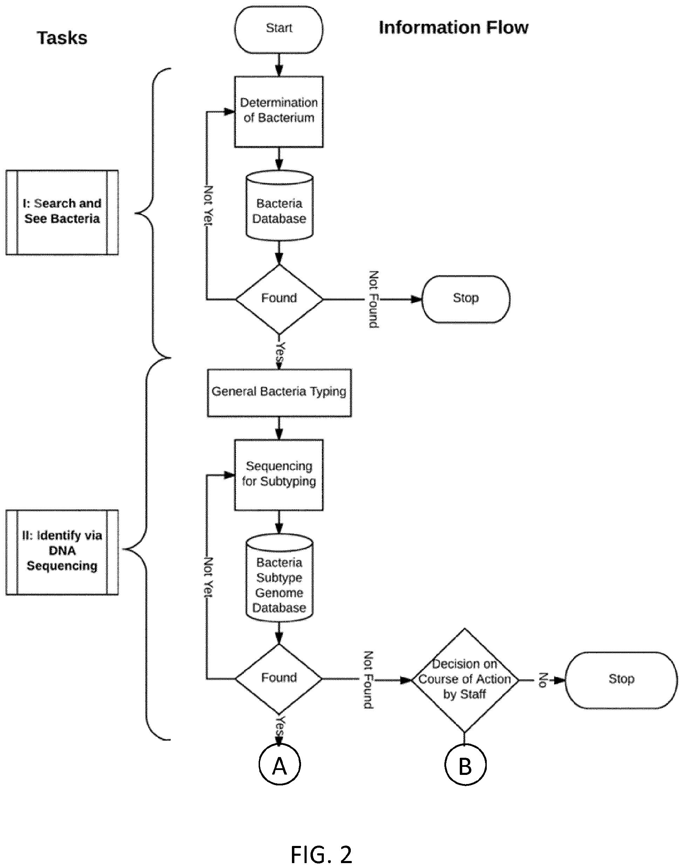

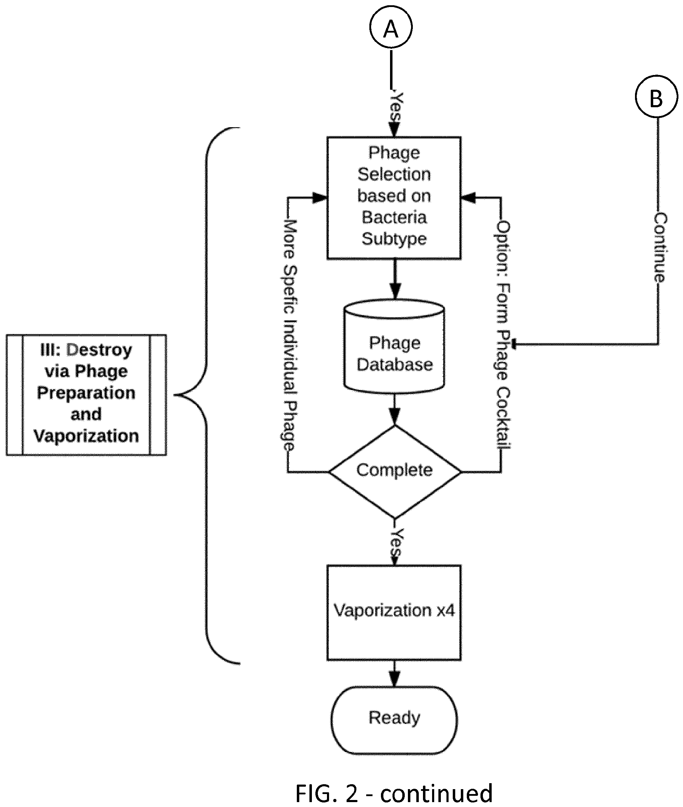

The nature of the disclosure is thus that the searching and scanning device is always directly linked to the detection system and the phage containers. A CPU unit, as described in Figure 2, is involved in data handling of the subsystems and in the decision-making of next phase actions.

### The Apparatus: Mobile and Static Embodiment

Device embodiments can vary from mobile to static depending on the circumstances wherein the device is requested to operate. One mobile embodiment of the disclosure, but not limited to this example, is a mobile robot with a scanning device. Such device was for the first time successfully used in the pioneering study of Adán et al. [2015] who, in their study for automatic creation of simple three-dimensional indoor models, used automatic scanning with mobile robots. In line with their proposed strategy to automate the complete indoor scanning process with the help of mobile robots their system of two agents (the scanner and the mobile robot) can be used along with their interaction process.

A static embodiment for the current disclosure, but not limited to this example, could be a system above a processing line where products are monitored and treated when necessary. However, common to all forms of embodiment is the aimed idea of automation. The device functions automatically and without any human intervention being necessary.

### Phase I: Scanning and Detection

Biochemical identification is the currently used approach to detect pathogens in the food and clinical industries. Although these could function as an early and general warning device the current disclosure proposes a faster and more recent technique in the field of detection. Scanning, with the SID system, is done with spectral devices equipped with pattern recognitions models that are pre-programmed to find general groups of bacteria. Techniques incorporated in the current disclosure are advanced spectral analysis techniques including MALDI-TOF mass spectrometry, hyperspectral imaging, RAMAN spectroscopy, and elastic light scatter (ELS) techniques. These optical devices are installed in a dome within the disclosure where they automatically scan surfaces for optical traces of pathogenic microbiological presence. The scanning device in the SID system provides an accessible way to quickly identify harmful bacteria by analysing the unique scattering patterns micro-organisms produce. By encoding the micro- and macro-structural morphology of a bacterial colony and then, aided by a spatial light modulator reading and decoding the scatter pattern of colonies
enables accurate bacterial identification. An example of a used detection device, but not limited to this is example, could be the usage of examination of forward light scattering on bacteria colonies in a Fourier transform optical system with converging spherical wave as presented by Buzalewic et all (2001). By analysing the bacteria colony diffraction signature as well as its Fresnel pattern a high accuracy determination can be achieved. Inspection functions with a light source that illuminates the object to be measured. To this object markers can be applied to enhance measurability, thereby improving the sensitivity of the measurement. These markers could also be, as e.g. described in DE102006009709, using bacteriophages themselves as a method to detect a microorganism or bacterium. These markers are dispersed through the vaporizer present on the system. Reflecting scatter-patterns are then read by observing the photon-colony interaction which wavefront characteristics differ based on the colons physical differences. Based on a model databank the diffraction signature can be related to previously measured micro-organisms. The real time reading of diffraction signatures provides a rapid method for a direct indication of the microbiological contamination of the measured object.

The spectral and biochemical measurements of this early phase are used to discriminate between the different kind of contaminants, as results will indicate which area is contaminated and for example which percentage of the surface is covered. This data will function as output for the CPU device that uses it to compute specific DNA sequencing action. After treatment (phase three) this first phase can be rerun to reveal if the contamination is still present.

The current disclosure complements this automatic searching and detection technique with the automated determining technique of DNA, RNA and/or mRNA sequencing. In anticipation of the sequencing phase, the vaporizer, is used to humidify the surfaces that are to be examined. Samples are provided to the sequencing device via a vacuum system that takes in droplets from the humidified surface and applies them on the sequencing unit. Based on the genome sequencing direct action can be taken, with a phage fogging device producing an aerosol which droplets contain carefully selected phages. So after the first phase, which is automatic continuous rapid measuring, there is the possibility of advanced determination that then starts the automatic elimination of the pathogenic contaminants present on either biological (food products, skin, wounds etc.) as well as non-biological surfaces (walls, machinery etc.).

### Phase II: Determination of Contaminating Microorganisms

After the spectral devices gave their indication of possible contaminants and humidified the areas that require closer examination, DNA, RNA and mRNA sequencing can be used to accurately identify and differentiate both viral and bacterial species present within or on the surface of the object measured. Determination is done with a portable real-time device for DNA and RNA sequencing. The most common would be classical PCR (Polymerase Chain Reaction), a technique based on using the ability of DNA polymerase to synthesize new strands of DNA complementary to the offered template strand. During the sequencing PCR process dideoxynucleotides are added to the reaction next to normal deoxynucleotides. Because DNA polymerase can add a nucleotide only onto a resisting 3'-OH group (a place for the new nucleotide to attach), and since a dideoxynucleotide lacks an OH-group, it needs a primer to which it can add the first nucleotide. This requirement makes it possible to delineate a specific region of the template sequence that the researcher wants to amplify. At the end of the PCR reaction, the specific sequence will be accumulated in billions of copies. This enables a detector to read base colour sequences and to accurately classify the present microorganism. This determination process in the current disclosure can also be done via whole-genome sequencing methods. These techniques are similar to PCR but are able to sequence more DNA simultaneously, making it more suitable for reading genomes. A possible determination method for the current disclosure, but not limited to this, is nanopore-based genomic sequencing wherein protein nanopores function as a means of differentiating nucleotides on a single strand of DNA. Via this way the sequence of both nucleic acid and protein can be determined. Recent studies have shown that with using whole-genome sequencing researchers were able to obtain rapidly precise species and clonal information as well as predicted antimicrobial susceptibility profiles. Direct sequencing, additionally, yielded information on multiple forms of bacterial presence thereby exceeding the possibilities of conventional techniques (Hasman et al., 2014). The current disclosure uses these determining techniques as an extension of the optical detection techniques. By investigating the contamination on DNA level with sequencing techniques the most complete and accurate determination can be given resulting in optimal information for the phage decontamination device.

### Phase III: The Elimination of Bacteria

Elimination of contaminating microorganisms is done with phage administration. This involves the use of specific viruses- viruses that can attack only bacteria - to kill pathogenic microorganisms. Knowledge of bacterial population densities, bacteria properties and environmental conditions are crucial in assuring the killing capacity of the phages since the successful elimination of a bacterial pathogen by a therapeutic phage or a cocktail of therapeutic phages can only occur when the phage-mediated bacterial cell killing at the infection site is faster than the rate of the bacterial cell number increase. After being informed by the DNA sequencing bacteria identification device the phage administration device can start the decontaminating process. Phages hold a strong possibility of killing pathogenic bacterial presence by their ability to destroy their bacterial hosts.

The current disclosure uses lytic phages in opposition to temperate phages. Where temperate phages can actually integrate their DNA into the host DNA the lytic phages always have to infect from the outside. They reprogram the host cell and release a burst of phage through breaking open, or lysing, the cell after a relatively fixed interval. The selection for lytic phages is motivated by the fact that temperate phages can become lysogenic where, by integrating their DNA with the host DNA, the prophage and the specific bacterium and all its progeny become virtually associated.

Using lytic phages in the current disclosure has the benefit that the phages are known to be rapidly cleared by the immune system. The fast dissolving proves its usability in biological settings. When host bacteria are present, lytic phages will exterminate these and then, when all pathogenic bacteria are exterminated, dissolve and be cleared by the immune system. Due to its self-destruction after extermination of the target bacteria, the use of lytic phages will leave no notable traces.

Phages operate by carrying genetic material between susceptible cells and then reproduce in those cells. However bacteriophages cannot infect cells of more complex organisms leaving them to target only specific kinds of bacterial cells. As Kutter summarizes the exterminating possibility of phages: "Tips op phage tails have the ability to bind to specific molecules on the surfaces of their target bacteria. The viral DNA is then ejected through the tail into the host cell, where it directs the production of progeny phages." Each strain of bacteria, has characteristic features such as its protein, carbohydrate, and lipopolysaccharide molecules. Although these molecules are involved in forming pores, motility, and binding of the bacteria to particular surfaces they also form a point for phage attachment. This means that every molecular characteristic of a bacterium functions as a receptor for particular phages. Different phages can attack many receptors on the same bacteria, indicating that a bacteria is never completely protected from phages since phages can act on different receptors.

For phages different administration techniques have been tested. Phages are versatile in application form, as liquid, cream etc. in addition to being suitable for most routes of administration. However in, for example, the medical sphere not many forms have been found to assure effectivity. Therefore the current disclosure preferably also consists of a unique form administration namely via a fogging device, administrating the phages on larger surfaces. The device releases the decontaminant that comprises at least one bacteriophage, known to propagate in bacteria of the bacterial species, as an aerosol with droplets of an average diameter of 0.2 - 40 micro-meter. The vaporization system can be sterilized by autoclaving, contains a fan for aerosol distribution, and is equipped with a sensor that provides feedback on the humidity in the room. Based on this feedback condensation is monitored and humidity is kept to a minimum. In for example decontaminating rooms with phages, it is found to be most effective when the room is incubated with the aerosol for a period between 1 and 600 seconds, a frequency between 2 and 60 times and intervals between 1 and 1800 seconds. The current disclosure makes use of the fact that different phages can be mixed as cocktails to broaden their properties, typically resulting in a collectively greater antibacterial spectrum of activity. Something which can easily be achieved with aerosols based on characteristics such as temperature and condensation degrees. The usage of aerosol enhances this extending possibility of phages by enabling it to penetrate into pits and other structures that are not easily accessible to regular cleaning methods. This, in combination with the fast expanding of the phages in the decontaminant, makes that phage usage is not limited to surfaces that are in direct contact with air.

Additional advantages of phage aerosols are that they are able to reach into problem areas and treat localized infections that are relatively inaccessible via the circulatory system and that they have the ability to clear at least some biofilms by actively penetrating their way into biofilms by lysing one bacterial layer at a time or due to the display of biofilm exopolymer-degrading depolymerases.

Therefore the current disclosure can be functional for countering the rising incidence of nosocomial infections and for combating bacteria that are resistant against most or all known antibiotics. The current disclosure could serve as a method in medicine for apt bacteria determination and elimination, having the advantage that immediate clarity is given about the nature of the infection and immediate treatment can be provided by phages that are far more effective than antibiotics in areas of the body where the circulation is bad. The disclosure can serve as infection treatment without the dysbiosis (the bacterial imbalance in the body) and its consequences (serious secondary infections involving relatively resistant bacteria) brought about by many antibiotic treatments.

For phage selection the current disclosure uses a method of rotating phage cocktail administration. This method of application overcomes the problems of bacterial resistance, since by the use of constantly shifting well-chosen mixtures of phages constantly different bacterial receptors are being targeted. The system contains multiple containers of decontaminants and based on computer selection the adequate phage is coupled to the administration device from a rotating phage container holder. By rotating, and varying the composition of the phage cocktail, the bacteria can still successfully be fought while at the same time preventing resistance to the treatment to occur.

When pathogenic bacteria are typed and monitored accurately chosen phages can be administered and the phages can achieve their full potentiality and effectivity. Phages are self-replicating but also self-limiting because they multiply only as long as sensitive bacteria are present. With the current disclosure phage administration can be used in various settings and for a wide spectrum of microbiological contamination. The combination of automatic contamination searching, accurate determination and apt elimination of contaminating microorganisms by using rotating phage cocktails results in a unique and powerful device applicable in many fields of research and work domains.

### Two Non-Limiting Embodiment Examples

The main application of this system will be in a clinical setting, where contact with contaminated surfaces result in significant healthcare costs and patient discomfort. An application of the SID system, but not limited to this application, in the hospital and healthcare environment is a mobile robot that automatically scans intensive-care units and decontaminates them overnight. More specifically the system comprises of a track-laying carrier vehicle upon which portable DNA sequencing unit, five phage containers, a vaporizer, a vacuum device, and a hyperspectral scanning device in a dome are installed. For detection of a major healthcare threat, Multi-resistant staphylococcus aureus (MRSA), a marker is vaporized in the habitat that enhances fluorescence of the MRSA bacterium. When the scanning system picks up and communicates traces of MRSA contamination in the habitat that is being examined or on the equipment present in said habitat, the vaporizer applies water to the contaminated surface and takes in these droplets. These droplets are placed on the portable DNA sequencer after which the exact genomic structure of the found bacterium is determined. Output of these findings (the exact serotype of the bacteria) functions as input for a computer system that calculates the optimal phage cocktail composition and prepares the phage cocktail for vaporization. The vaporization process is started, the exact procedure (such as intervals and frequency of vaporization) being dependent on the habitat that is to be decontaminated. After a certain amount of time, equally dependent on the characteristics of the habitat, the optical scanning is rerun to conclude if the decontamination was successful. If the contamination has been erased the vehicle proceeds to the next location.

An example of a static system using the SID method, but not limited to this example, could be an apparatus used for the detection and elimination of the pathogenic E.coli bacteria (one of the most frequent food-borne pathogens) placed over a food processing line. Although not all E.coli's are bad there is a serotype of E.coli that causes approximately 100,000 illnesses, 3000 hospitalizations and 90 deaths annually in the United States alone: Shiga toxin-producing E.coli (STEC). The most notorious E. coli bacteria that produces Shiga Toxin is E.coli O157: H7. This STEC is subject of much concern, due to its contamination of such products as hamburgers and unpasteurized fruit juices leading to serious problems.

Particularly in young children and the elderly, deaths have occurred from haemorrhagic colitis and from haemolytic-uremic syndrome. Antibiotic therapy has shown no benefit; it is actually generally contraindicated because it leads to increased toxin release. This E.coli alone is estimated to cost the United States about 800 million annually. The pathogenic bacteria is often found in contaminated manufactured food- or drinking- products. The FDA and FSIS state that in the event that pathogenic E. coli is found in a food product a Class I recall is initiated, indicating the highest possible degree of hazard such as serious health problems or death. The phage for this pathogen, that haunts food industry and creates massive recalls for industries from meat packers to apple juice producers, turns out to be T4-like and to infect virtually all tested 0157 strains.

The current disclosure could operate thusly in the prevention of E.coli infections: Using the presented method for bacteria determination and elimination as presented above a possible application, but not limited to this application, could be envisioned as follows/as described in figure 1. Food products on a processing line are individually and optically checked for contamination, defined as the presence of an unwanted substance, with spectral optical measurements that runs two analyses on the product: it compares the present image to an existing food-model thereby actively controlling the product for deviations with images of the ideal product. In case deviations are found the characteristics of the contaminant are run against a database of contaminants resulting in a match with known groups of bacteria. The product is placed on a parallel line after which DNA sequencing, either via human sample preparation or via automated sample preparation, is used to further investigate the found contamination. When the present microbiological contaminant is found to be the pathogenic Shiga toxin-producing E.coli, this is communicated to the decontaminating phage device. This is a system where phage cocktails of various make-up are stored. By being linked to the measuring system the system communicates what strain of pathogenic E.coli, to continue the example, is present on the product and what receptors to target with the available phages. For E.coli, using latest research as suggested by Zuber et al. 2007, phage therapy ranges further than the normally applied T4 phages and has optimal coverage of pathogenic E.coli strains when a new branch of T-even phages is added. This results in a phage cocktail consisting of T4-, RB69-, JS98-, and RB49-like phages additionally complimented with CEH-162 coliphage, being applied in the form of spray ( PFU/ml). This results in a uncontaminated and above all safe product since after lysing the bacteria, no viral stains remain on the product. Also the chance that there will be an exchange between both the bacterial host genome and other phage genomes is very small due to the fact that the bacterial genome is rapidly and completely degraded in an early infection stage. After the phage vaporizer applies the necessary amount of phages on the product, the product after treatment is run again through the spectral imaging installation to be sure that all pathogenic E.coli's have successfully been eliminated. The disclosure excels in specificity and speed while performing an 100% check of the food products in line during the production process. It not only leaves chances of contaminated products distributed to supermarkets or to consumers negligible, it also offers a means reprocess treated products. Where contaminated products now are taken out of production resulting in entire badges of products being destroyed when contamination is found, the current disclosure provides a way to achieve an organic and ecological solution to contaminated products. This allows for sustainable production. The overall example shows how safety guarantees are optimized and waste of products is minimalized by interfering only with contaminated products. Lytic phages, in the current literature on the topic rendered harmless without bacterial host cells, can optimally be applied due to the added discriminative detection method.

### Future implications and Fields of Appliance

From this example it becomes apparent that possibilities exceed the food and clinical industry. Although especially in the field of medicine accurate administration of the right phage will be of crucial importance even beyond the Western world applications are thinkable. E.coli is also responsible for one third of the dysentery cases in underdeveloped countries leaving possibilities for its detection and elimination to go far beyond the production controlled example above.

## Claims

1. A system to rapidly search, identify and eliminate microbiological contamination on biological and non-biological surfaces comprising the following devices in combination:
A) A detection device able to provide data output for a general or early warning, said detection device being a spectral device equipped with pattern recognitions models that are pre-programmed to find general groups of bacteria.
B) A DNA, RNA or mRNA sequencing device for genomic identification of organisms present on measured surface.
C) A decontamination device comprising:
C1 - Multiple decontaminant containers, each containing a decontaminant that comprises of at least one lytic bacteriophage.
C2 - A device for administration of phages comprising administration means.
D) Connecting means for connecting the above devices to a CPU unit wherein:
- The output generated by the detection device serves as functional input for the identification device.
- The output generated by the identification device serves functional input for the decontamination device.
E) A CPU unit comprising means for micro-biological spectral pattern recognition; DNA, RNA or mRNA sequencing analysis tools; a phage database; phage selection means.

2. A system according to claim 1, being a mobile system.

3. A system according to any of the previous claims, wherein the device for administration of phages is a vaporizer for producing an aerosol with droplets of an average diameter of 0.2 - 40 micrometre that comprises at least one phage.

4. A system according to claim 3, where aerosol droplets have an average diameter of 1 - 8 micrometre.

5. A system according to claim 3 or 4, wherein the vaporizer can be sterilized by autoclaving and/or comprises a fan for distributing the aerosol in a room .

6. A system according to any of the previous claims, wherein the DNA, RNA or mRNA sequencing device comprises a vacuum unit for taking in water droplets containing contaminants.

7. A system according to any of the previous claims, comprising a rotating tray for holding the multiple decontaminant containers.

8. A method for apt bacteria detection, identification and elimination using the apparatus of claim 1, working in the following phases:
- Phase I: See. Perform a surface scan on biological and/or non-biological surfaces by using optical means. Analysing the spectral patterns reflected to determine whether microbiological contamination is present on the measured surface by using a CPU unit.
- Phase II: Identify. Via DNA, RNA or mRNA sequencing methods identifying of characteristic molecules to determine within each subclass of bacteria the exact serotype of the bacteria present on the examined surface by using DNA, RNA or mRNA sequencing means and a CPU unit.
- Phase III: Destroy. Communicating characteristics of the found bacteria back to the CPU unit which calculates and selects the adequate cocktail of phages for decontamination followed by automatic administration of the apt phages on the contaminated parts of the surface.

9. A method as described in claim 8, where detection, identification, and elimination takes place fully automatic.

10. A method as described in any of the above claims, where the system of phage-administration makes use of an aerosol which droplets contain said phage.

11. A method as described in claim 8, where the decontaminant is released in the form of an aerosol with droplets of an average diameter of 0.2 - 40 micrometre, of which droplets contain at least one phage that is known to propagate in bacteria of the bacterial species, for use in removing bacteria of said bacterial species.

12. A method according to any of claims 10 to 11, comprising incubating a room with the aerosol for a period between 1 and 600 seconds, a frequency between 2 and 60 times and intervals between 1 and 1800 seconds.

## Patentansprüche

1. Ein System zum schnellen Suchen, Identifizieren und Beseitigen von mikrobiologischer Kontamination auf biologischen und nicht-biologischen Oberflächen, das die folgenden Vorrichtungen in Kombination umfasst:
A)- eine Nachweisvorrichtung, die in der Lage ist, Datenausgabe für eine allgemeine oder frühzeitige Warnung bereitzustellen, wobei es sich bei der Nachweisvorrichtung um eine Spektralvorrichtung handelt, die mit Mustererkennungsmodellen ausgestattet ist, die vorprogrammiert sind, um allgemeine Bakteriengruppen zu finden.
B)- eine DNA-, RNA- oder mRNA-Sequenziervorrichtung zur genomischen Identifizierung von Organismen, die auf einer gemessenen Oberfläche vorhanden sind.
C)- eine Dekontaminationsvorrichtung, umfassend:
C1- mehrere Dekontaminationsmittelbehälter, die jeweils ein Dekontaminationsmittel enthalten, das aus mindestens einem lytischen Bakteriophagen besteht.
C2- eine Vorrichtung zur Verabreichung von Phagen, die Verabreichungsmittel umfasst.
D)- Verbindungsmittel zum Verbinden der obigen Vorrichtungen mit einer CPU-Einheit, wobei:
- die von der Nachweisvorrichtung erzeugte Ausgabe als funktionelle Eingabe für die Identifizierungsvorrichtung dient.
- die von der Identifizierungsvorrichtung erzeugte Ausgabe als funktionelle Eingabe für die Dekontaminationsvorrichtung dient.
E)- eine CPU-Einheit, umfassend Mittel zur mikrobiologischen Spektralmustererkennung; DNA-, RNA- oder mRNA-Sequenzierungsanalysewerkzeuge; eine Phagen-Datenbank; Phagen-Auswahlmittel.

2. Ein System nach Anspruch 1, wobei es sich um ein mobiles System handelt.

3. Ein System nach einem der vorstehenden Ansprüche, wobei es sich bei der Vorrichtung zur Verabreichung von Phagen um einen Zerstäuber handelt zum Erzeugen eines Aerosols mit Tröpfchen eines durchschnittlichen Durchmessers von 0,2 - 40 Mikrometer, das mindestens einen Phagen umfasst.

4. Ein System nach Anspruch 3, wobei Aerosoltröpfchen einen durchschnittlichen Durchmesser von 1 - 8 Mikrometer aufweisen.

5. Ein System nach Anspruch 3 oder 4, wobei der Zerstäuber durch Autoklavieren sterilisiert werden kann und/oder einen Lüfter zum Verteilen des Aerosols in einem Raum umfasst.

6. Ein System nach einem der vorstehenden Ansprüche, wobei die DNA-, RNA- oder mRNA-Sequenziervorrichtung eine Vakuumeinheit zum Aufnehmen von Kontaminanten enthaltenden Wassertröpfchen umfasst.

7. Ein System nach einem der vorstehenden Ansprüche, das eine Drehplatte zum Halten der mehreren Dekontaminationsmittelbehälter umfasst.

8. Ein Verfahren zum Nachweis, zur Identifizierung und Beseitigung von APT-Bakterien unter Verwendung der Einrichtung nach Anspruch 1, das in den folgenden Phasen abläuft:
- Phase I: Sehen. Durchführen eines Oberflächen-Scans an biologischen und/oder nicht-biologischen Oberflächen unter Verwendung von optischen Mitteln. Analysieren der reflektierten Spektralmuster unter Verwendung einer CPU-Einheit, um zu bestimmen, ob mikrobiologische Kontamination auf der gemessenen Oberfläche vorhanden ist.
- Phase II: Identifizieren. Identifizieren von charakteristischen Molekülen über DNA-, RNA- oder mRNA-Sequenzierverfahren, um innerhalb jeder Unterklasse von Bakterien den genauen Serotyp der auf der untersuchten Oberfläche vorhandenen Bakterien unter Verwendung von DNA-, RNA- oder mRNA-Sequenziermitteln und einer CPU-Einheit zu bestimmen.
- Phase III: Vernichten. Rückmelden von Charakteristiken der gefundenen Bakterien an die CPU-Einheit, die den geeigneten Phagen-Cocktail zur Dekontamination berechnet und auswählt, gefolgt von der automatischen Verabreichung der APT-Phagen an die kontaminierten Teile der Oberfläche.

9. Ein Verfahren wie in Anspruch 8 beschrieben, wobei der Nachweis, die Identifizierung und Beseitigung vollautomatisch stattfinden.

10. Ein Verfahren wie in einem der obigen Ansprüche beschrieben, wobei das Phagenverabreichungssystem ein Aerosol verwendet, dessen Tröpfchen den Phagen enthalten.

11. Ein Verfahren wie in Anspruch 8 beschrieben, wobei das Dekontaminationsmittel in Form eines Aerosols mit Tröpfchen eines durchschnittlichen Durchmessers von 0,2 - 40 Mikrometer freigesetzt wird, von dem Tröpfchen mindestens einen Phagen enthalten, von dem bekannt ist, dass er sich in Bakterien der Bakterienspezies vermehrt, zur Verwendung beim Entfernen von Bakterien der Bakterienspezies.

12. Ein Verfahren nach einem der Ansprüche 10 bis 11, das das Inkubieren eines Raums mit dem Aerosol über einen Zeitraum zwischen 1 und 600 Sekunden, einer Frequenz zwischen 2 und 60 Mal und Intervallen zwischen 1 und 1800 Sekunden umfasst.

## Revendications

1. Un système pour rechercher, identifier et éliminer rapidement la contamination microbiologique sur les surfaces biologiques et non biologiques comprenant les dispositifs suivants en combinaison
A) Un dispositif de détection capable de fournir des données de sortie pour une alerte générale ou précoce, ledit dispositif de détection étant un dispositif spectral équipé de modèles de reconnaissance de motifs préprogrammés pour trouver des groupes généraux de bactéries.
B) Un dispositif de séquençage d'ADN, d'ARN ou d'ARNm pour l'identification génomique des organismes présents sur la surface mesurée.
C) Un dispositif de décontamination comprenant :
C1 - Plusieurs conteneurs de décontaminants, chacun contenant un décontaminant qui comprend au moins un bactériophage lytique.
C2 - Un dispositif pour l'administration de phages comprenant des moyens d'administration.
D) Des moyens de connexion pour connecter les dispositifs ci-dessus à une unité CPU dans laquelle
• La sortie générée par le dispositif de détection sert d'entrée fonctionnelle pour le dispositif d'identification.
• La sortie générée par le dispositif d'identification sert d'entrée fonctionnelle pour le dispositif de décontamination.
E) Une unité CPU comprenant des moyens de reconnaissance de motifs spectraux micro-biologiques; des outils d'analyse de séquençage d'ADN, d'ARN ou d'ARNm ; une base de données de phages ; des moyens de sélection de phages.

2. Un système selon la revendication 1, étant un système mobile.

3. Un système selon l'une quelconque des revendications précédentes, dans lequel le dispositif pour l'administration de phages est un vaporisateur pour produire un aérosol avec des gouttelettes d'un diamètre moyen de 0.2 - 40 micromètres qui comprend au moins un phage.

4. Un système selon la revendication 3, où les gouttelettes d'aérosol ont un diamètre moyen de 1 - 8 micromètres.

5. Un système selon la revendication 3 ou 4, dans lequel le vaporisateur peut être stérilisé par autoclavage et/ou comprend un ventilateur pour distribuer l'aérosol dans une pièce.

6. Un système selon l'une quelconque des revendications précédentes, dans lequel le dispositif de séquençage d'ADN, d'ARN ou d'ARNm comprend une unité à vide pour aspirer des gouttelettes d'eau contenant des contaminants.

7. Un système selon l'une quelconque des revendications précédentes, comprenant un plateau tournant pour contenir les multiples conteneurs de décontaminants.

8. Une méthode pour la détection, l'identification et l'élimination aptes des bactéries en utilisant l'appareil de la revendication 1, travaillant dans les phases suivantes :
• Phase I : Voir. Effectuer une analyse de surface sur des surfaces biologiques et/ou non biologiques en utilisant des moyens optiques. Analyser les motifs spectraux réfléchis pour déterminer si une contamination microbiologique est présente sur la surface mesurée en utilisant une unité CPU.
• Phase II : Identifier. Via des méthodes de séquençage d'ADN, d'ARN ou d'ARNm, identifier des molécules caractéristiques pour déterminer au sein de chaque sous-classe de bactéries le sérotype exact des bactéries présentes sur la surface examinée en utilisant des moyens de séquençage d'ADN, d'ARN ou d'ARNm et une unité CPU.
• Phase III : Détruire. Communiquer les caractéristiques des bactéries trouvées à l'unité CPU qui calcule et sélectionne le cocktail adéquat de phages pour la décontamination suivie de l'administration automatique des phages aptes sur les parties contaminées de la surface.

9. Une méthode telle que décrite à la revendication 8, où la détection, l'identification et l'élimination se déroulent entièrement automatiquement.

10. Une méthode telle que décrite dans l'une quelconque des revendications précédentes, où le système d'administration de phages fait usage d'un aérosol dont les gouttelettes contiennent ledit phage.

11. Une méthode telle que décrite à la revendication 8, où le décontaminant est libéré sous forme d'un aérosol avec des gouttelettes d'un diamètre moyen de 0.2 - 40 micromètres, dont les gouttelettes contiennent au moins un phage qui est connu pour se propager dans les bactéries de l'espèce bactérienne, pour utilisation dans l'élimination des bactéries de ladite espèce bactérienne.

12. Une méthode selon l'une quelconque des revendications 10 à 11, comprenant l'incubation d'une pièce avec l'aérosol pendant une période entre 1 et 600 secondes, une fréquence entre 2 et 60 fois et des intervalles entre 1 et 1800 secondes.
